# EUROPEAN PATENT APPLICATION

(11) **EP 4 825 351 A1**
(43) Date of publication of application: **21.10.2026**
(21) Application number: 25776979.4
(22) Date of filing: 24.03.2025
(51) Int. Cl.: A61F 13/49, A61F 13/496, A61F 13/532, A61F 13/533

(54) **ABSORBENT ARTICLE**

(30) Priority: 25.03.2024 JP 2024048789
(71) Applicant: Unicharm Corporation, Ehime 799-0111 (JP)
(72) Inventor: NARA, Yuki, Kanonji-shi, Kagawa 769-1602 (JP); UTSUNOMIYA, Hideto, Kanonji-shi, Kagawa 769-1602 (JP)
(74) Representative: Staeger & Sperling Partnerschaftsgesellschaft mbB
(86) International application number: PCT/JP2025/011421
(87) International publication number: WO 2025/205586

(57) **Abstract**

An absorbent article (1) having an absorbent core (11), wherein: the absorbent article (1) has a crotch elastic member (40) that expands and contracts in the longitudinal direction; at least a part of the crotch elastic member (40) is provided at a central part in the left-right direction of a crotch part, which is the central region when the absorbent article (1) is divided into three equal parts in the longitudinal direction; at the central position of the absorbent article (1) in the longitudinal direction, the absorbent core (11) is a single layer; and, when a lifting test is performed after liquid is dropped using the central position in the left-right direction at a two-folded position in the longitudinal direction as a dripping position, the lifting test involving gripping and lifting both waist-side ends in the left-right direction of the absorbent article (1) in a longitudinally two-folded state, the central part in the left-right direction of the lower end of the absorbent article (1) forms a convex shape toward the upper side.

## Description

### FIELD

The present invention relates to an absorbent article.

### BACKGROUND

Patent Literature 1 discloses an absorbent article (wearable article), specifically an incontinence underpants-shaped diaper in which a first elastic member is arranged substantially at a widthwise central portion of the absorbent article so as to stretch along the longitudinal direction of the absorbent article. The first elastic member improves close contact between an excretion region (crotch portion) and an absorbent body, thereby suppressing leakage of excreted fluid along the wearer's skin to the outside even in cases where the urine flow rate is low.

### [CITATION LIST]

### [PATENT LITERATURE]

Patent Literature 1: Japanese Patent No. 5756093

### SUMMARY

### [TECHNICAL PROBLEM]

Patent Literature 1 discloses an absorbent body having a two-layer structure, the absorbent body in which a first absorbent-body layer and a second absorbent-body layer overlap with each other at a longitudinal central position that comes into contact with the wearer's crotch portion. In the two-layer portion, while a sufficient amount of excreted fluid can be absorbed, the thickness increases. Thus, the wearer may feel discomfort due to stiffness. Furthermore, when the absorbent body absorbs excreted fluid, the absorbent body sags due to its increased weight. This may reduce close contact of the absorbent body with the wearer's crotch portion, thereby making the wearer likely to feel discomfort. In Patent Literature 1, leakage occurring in cases where the urine flow rate is low and cases where the amount of urine is large is addressed as a problem, but deformation of the absorbent body after excretion is not taken into consideration. In particular, in an absorbent body having a two-layer structure, when the first absorbent-body layer and the second absorbent-body layer have different widths, boundaries between the single-layer portion and the two-layer portion are located at both widthwise side portions of the absorbent body, and such boundaries are likely to serve as starting points for bending of the absorbent body. Therefore, after the absorbent body absorbs excreted fluid, even with the first elastic member, the absorbent body cannot be brought into close contact with the wearer's crotch portion, and the absorbent body may sag.

The present invention has been made in light of conventional problems such as that described above, and an aspect of the present invention is to provide an absorbent article that suppresses leakage of excreted fluid while also suppressing discomfort caused by stiffness at the wearer's crotch portion and by reduced close contact with the crotch portion after excretion.

### [SOLUTION TO PROBLEM]

A main aspect of the present invention for achieving the above-described aspect is an absorbent article having, in an unfolded and stretched state, a longitudinal direction, a lateral direction, and a thickness direction that intersect one another, the absorbent article including: an absorbent core; and a crotch elastic member that stretches and contracts in the longitudinal direction, at least a part of the crotch elastic member being provided in a lateral central portion of a crotch portion, the crotch portion being a central region obtained by dividing the absorbent article into three equal regions in the longitudinal direction, at a longitudinal central position of the absorbent article, the absorbent core being a single layer, when a lifting test is performed, a lateral central portion at a lower end of the absorbent article forming an upwardly convex shape, the lifting test being a test in which, after liquid is dropped onto a dropping position that is a lateral central position of a fold position for folding the absorbent article one time in the longitudinal direction, two lateral end portions of a waist-side end portion of the absorbent article in a longitudinally folded state are gripped and lifted.
Features of the present invention other than the above will become clear by reading the description of the present specification with reference to the accompanying drawings.

### [ADVANTAGEOUS EFFECT OF THE INVENTION]

According to the present invention, it is possible to provide an absorbent article that suppresses leakage of excreted fluid while also suppressing discomfort caused by stiffness at the wearer's crotch portion and by reduced close contact with the crotch portion after excretion.

### BRIEF DESCRIPTION OF DRAWINGS

FIG. 1 is a schematic front view of an underpants-shaped disposable diaper 1.
FIG. 2 is a schematic plan view of the diaper 1 in an unfolded and stretched state.
FIG. 3 is a schematic cross-sectional view taken along a line C-C in FIG. 2.
FIG. 4 is a schematic cross-sectional view of an absorbent main body taken along a line CL-CL in FIG. 2.
FIG. 5 is a view illustrating the arrangement of crotch elastic members 40.
FIG. 6 is an illustrative view of a lifting test.
FIG. 7 is an illustrative view of the lifting test.
FIG. 8 is an illustrative view of the lifting test.
FIG. 9 is an illustrative view of a pre-absorption lifting test.
FIG. 10 illustrates a state in which a waist portion is stretched after the lifting test.
FIG. 11 is an illustrative view of a diaper 1 according to a modified example.
FIG. 12 is an illustrative view of compressed portions 60.
FIGS. 13A and 13B are illustrative views of the compressed portions 60.
FIGS. 14A and 14B are schematic cross-sectional views of an absorbent core 11.

### [DESCRIPTION OF THE EMBODIMENTS]

At least following matters will become clear with description of this specification and attached drawings.

Aspect 1 is an absorbent article having, in an unfolded and stretched state, a longitudinal direction, a lateral direction, and a thickness direction that intersect one another, the absorbent article including: an absorbent core; and a crotch elastic member that stretches and contracts in the longitudinal direction, at least a part of the crotch elastic member being provided in a lateral central portion of a crotch portion, the crotch portion being a central region obtained by dividing the absorbent article into three equal regions in the longitudinal direction, at a longitudinal central position of the absorbent article, the absorbent core being a single layer, when a lifting test is performed, a lateral central portion at a lower end of the absorbent article forming an upwardly convex shape, the lifting test being a test in which, after liquid is dropped onto a dropping position that is a lateral central position of a fold position for folding the absorbent article one time in the longitudinal direction, two lateral end portions of a waist-side end portion of the absorbent article in a longitudinally folded state are gripped and lifted.

According to Aspect 1, the crotch elastic member allows the absorbent core to be brought into close contact with the wearer's crotch portion, thereby suppressing leakage of excreted fluid. In addition, since the absorbent core is a single layer at a position that comes into contact with the wearer's crotch portion, the wearer is less likely to feel discomfort caused by stiffness. Due to the lifting test, deformation of the absorbent core after excretion is also taken into consideration, and thus, even after excretion, the state in which the absorbent core is in close contact with the wearer's crotch portion is likely to be maintained. Thus, wearer's discomfort caused by a decrease in close contact with the wearer's crotch portion (sagging of the absorbent article) can be reduced.

Aspect 2 is an absorbent article according to Aspect 1, wherein, in an unfolded and stretched state, a length of a stretchable portion of the crotch elastic member located on a front side with respect to the longitudinal central position of the absorbent article is longer than a length of a stretchable portion of the crotch elastic member located on a back side with respect to the longitudinal central position.

According to Aspect 2, the crotch elastic member is likely to be located at a position corresponding to the urethral opening of the wearer, and the crotch elastic member can directly push up the absorbent core toward the urethral opening. Thus, the absorbent core is likely to come into close contact with the urethral opening, and leakage of excreted fluid can be further suppressed.

Aspect 3 is an absorbent article according to Aspect 1 or 2, wherein, in an unfolded and stretched state, the stretchable portion of the crotch elastic member is arranged so as to span the longitudinal central position of the absorbent article.

According to Aspect 3, the crotch elastic member can directly push up the absorbent core at the longitudinal central position. Accordingly, in the lifting test, the lateral central portion at the lower end of the absorbent article can form an upwardly convex shape.

Aspect 4 is an absorbent article according to any one of Aspects 1 to 3, wherein the absorbent article includes a lateral elastic member that stretches and contracts in the lateral direction, in an unfolded and stretched state, a region delimited by the crotch elastic member is referred to as a crotch elastic region, and when the absorbent article is viewed in the thickness direction, the crotch elastic region does not overlap a stretchable portion of the lateral elastic member.

According to Aspect 4, it is possible to prevent the longitudinal elastic force of the crotch elastic member from being inhibited by the lateral elastic force of the lateral elastic member. Therefore, even after excretion, the crotch elastic member can push up the absorbent core, so that, in the lifting test, the lateral central portion at the lower end of the absorbent article can form an upwardly convex shape.

Aspect 5 is an absorbent article according to any one of Aspects 1 to 4, wherein the absorbent article includes a plurality of continuous elastic members that stretch and contract in the lateral direction and are continuous at a position overlapping the absorbent core with respect to the lateral direction, in an unfolded and stretched state, the continuous elastic member closest to the crotch elastic member on the front side in the longitudinal direction is referred to as a front-side continuous elastic member, the continuous elastic member closest to the crotch elastic member on the back side in the longitudinal direction is referred to as a back-side continuous elastic member, and in the longitudinal direction, a length from a front-side end of the stretchable portion of the crotch elastic member to the front-side continuous elastic member is shorter than a length from a back-side end of the stretchable portion of the crotch elastic member to the back-side continuous elastic member.

According to Aspect 5, on the front side of the absorbent article, the length of the region where the force of the elastic member does not directly act on the absorbent core is reduced. Therefore, the absorbent core is likely to be brought into close contact with the wearer's urethral opening located on the front side. This can further suppress leakage of excreted fluid.

Aspect 6 is an absorbent article according to any one of Aspects 1 to 5, wherein the absorbent article includes a continuous elastic member that stretches and contracts in the lateral direction and is continuous at a position overlapping the absorbent core with respect to the lateral direction, the crotch elastic member comprises a plurality of string-like elastic members, in an unfolded and stretched state, the continuous elastic member closest to the crotch elastic member on the back side in the longitudinal direction is referred to as a back-side continuous elastic member, and a force required to stretch a stretchable portion of the back-side continuous elastic member by a predetermined length in the lateral direction is smaller than an average value of a force required to stretch each of the plurality of string-like elastic members by a predetermined length in the longitudinal direction.

According to Aspect 6, due to the relatively weak elastic force of the back-side continuous elastic member, a space is formed between the wearer's buttocks and the absorbent core, and feces or a large amount of excreted urine can be temporarily accommodated in this space. This can suppress leakage of excrement. On the other hand, due to the relatively strong elastic force of the crotch elastic member, in the lifting test, the lateral central portion at the lower end of the absorbent article can form an upwardly convex shape.

Aspect 7 is an absorbent article according to any one of Aspects 1 to 6, wherein at the longitudinal central position of the absorbent article, a low-basis-weight region in which a basis weight of the absorbent core is lower than that in a surrounding region is not present.

According to Aspect 7, even when the absorbent core has a single-layer structure at the longitudinal central position, the absorbent core can ensure the absorption amount of excreted fluid. Thus, leakage of excreted fluid can be suppressed. In addition, in the lifting test, the risk of unintended deformation can be reduced, and, the lateral central portion at the lower end of the absorbent article can form an upwardly convex shape.

Aspect 8 is an absorbent article according to any one of Aspects 1 to 7, wherein the absorbent article includes a compressed portion in which the absorbent core is compressed in the thickness direction, the crotch elastic member comprises a plurality of string-like elastic members, and the compressed portion is arranged between the plurality of string-like elastic members arranged in the lateral direction.

According to Aspect 8, stiffness of the absorbent core between the crotch elastic members (string-like elastic members) is increased. Therefore, even after excretion, in conjunction with the absorbent core directly pushed up by the crotch elastic members, the absorbent core between the crotch elastic members is also likely to be pushed up. Accordingly, the absorbent core can be brought into close contact with the wearer's crotch portion in a surface-contact manner, thereby reducing wearer's discomfort caused by a decrease in close contact.

Aspect 9 is an absorbent article according to any one of Aspects 1 to 8, wherein the absorbent article includes a compressed portion in which the absorbent core is compressed in the thickness direction, and in an unfolded and stretched state, a region delimited by the crotch elastic member is referred to as a crotch elastic region, and in the crotch elastic region, the compressed portion includes at least a part of an inclined compressed portion extending along a direction inclined relative to the longitudinal direction and the lateral direction.

According to Aspect 9, the inclined compressed portion serves as a starting point for bending, and the absorbent core overlapping the crotch elastic region is likely to deform. Therefore, in the lifting test, the lateral central portion at the lower end of the absorbent article can form an upwardly convex shape.

Aspect 10 is an absorbent article according to Aspect 9, wherein the inclined compressed portion includes a plurality of first inclined portions and a plurality of second inclined portions, the plurality of first inclined portions extending along a first inclined direction, the plurality of second inclined portions extending along a second inclined direction that intersects the first inclined direction, the plurality of first inclined portions are arranged at intervals in the second inclined direction, the plurality of second inclined portions are arranged at intervals in the first inclined direction, and in an unfolded and stretched state, a lateral length of a region delimited by the plurality of first inclined portions and the plurality of second inclined portions is shorter than a lateral length of the crotch elastic region.

According to Aspect 10, the region delimited by the plurality of first inclined portions and the plurality of second inclined portions is more likely to be located within a portion of the absorbent core overlapping the crotch elastic region. Therefore, the inclined compressed portions that define the region serve as the starting points for bending, and the absorbent core overlapping the crotch elastic region is likely to deform. As a result, in the lifting test, the lateral central portion at the lower end of the absorbent article can form an upwardly convex shape.

Aspect 11 is an absorbent article according to any one of Aspects 1 to 10, wherein when a pre-absorption lifting test is performed, a lateral central portion at a lower end of the absorbent article forms an upwardly convex shape, the pre-absorption lifting test being a test in which the two lateral end portions of the waist-side end portion of the absorbent article that is in a dry state and a longitudinally folded state are gripped and lifted, and a height of the convex shape in the lifting test is greater than a height of the convex shape in the pre-absorption lifting test.

According to Aspect 11, before excretion, the absorbent core comes into close contact with the wearer's crotch portion, and thus, leakage at the time of first excretion can be suppressed. Even after excretion, the state in which the lateral central portion of the absorbent core is in close contact with the wearer's crotch portion is maintained. Thus, wearer's discomfort can be reduced.

Aspect 12 is an absorbent article according to any one of Aspects 1 to 11, wherein the absorbent article is an underpants-shaped absorbent article including a waist elastic member that stretches and contracts in the lateral direction, when the lifting test is performed, a waist-side end of the absorbent article forms a recessed shape downwardly curved, and a height of the convex shape is smaller than a vertical length from a virtual line connecting left and right ends at the waist-side end of the absorbent article to a lateral central portion of the waist-side end.

According to Aspect 12, after excretion, the lateral central portion at the lower end of the absorbent article forms a convex shape, while the height thereof is appropriately suppressed. Therefore, after excretion, while the absorbent core is brought into close contact with the wearer's crotch portion, it is possible to prevent an excessive pressing against the wearer's crotch portion, and to reduce wearer's discomfort.

Aspect 13 is an absorbent article according to any one of Aspects 1 to 12, wherein the absorbent article is an underpants-shaped absorbent article including a waist elastic member that stretches and contracts in the lateral direction, and after the lifting test is performed, in a state in which a gripping position of the waist-side end is widened to a length that is 80% of a length when the waist-side end of the absorbent article is maximally stretched in the lateral direction, a vertical length from a virtual line connecting left and right ends at the waist-side end of the absorbent article to a lateral central portion of the waist-side end is smaller than a height of the convex shape.

According to Aspect 13, in the worn state in which the waist portion of the absorbent article is stretched, the difference in the vertical direction between the lateral side portions and the central portion at the waist-side end is reduced. Therefore, the absorbent article can uniformly cover the entire waist of the wearer. On the other hand, since the lateral central portion at the lower end of the absorbent article forms an upwardly convex shape, even after excretion, the absorbent core can be brought into close contact with the wearer's crotch portion, thereby reducing the wearer's discomfort.

Aspect 14 is an absorbent article according to any one of Aspects 1 to 13, wherein the absorbent article includes an absorbent main body including the absorbent core and an exterior member, and the exterior member includes a pair of waist portions and a crotch portion that connects the pair of waist portions.

According to Aspect 14, the absorbent core that has increased in weight after excretion, is pulled upward by the crotch portion of the exterior member that is continuous from the waist portion. Thus, discomfort of the wearer caused by sagging of the absorbent core can be reduced.

### Embodiments

Hereinafter, an underpants-shaped absorbent article according to an embodiment of the present invention will be described with an underpants-shaped disposable diaper for adults (hereinafter also referred to as a "diaper 1") as an example. However, the absorbent article according to the present invention is not limited thereto, and is also applicable to tape-type disposable diapers, sanitary panties, and the like. A wearer of the absorbent article may be an infant, an animal, or other living creatures.

### Basic Configuration of Underpants-shaped Disposable Diaper 1

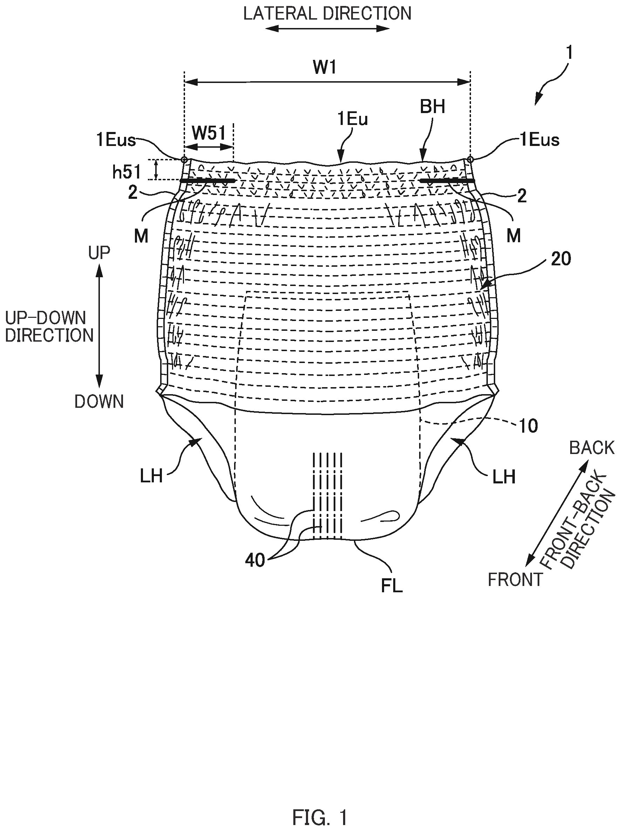

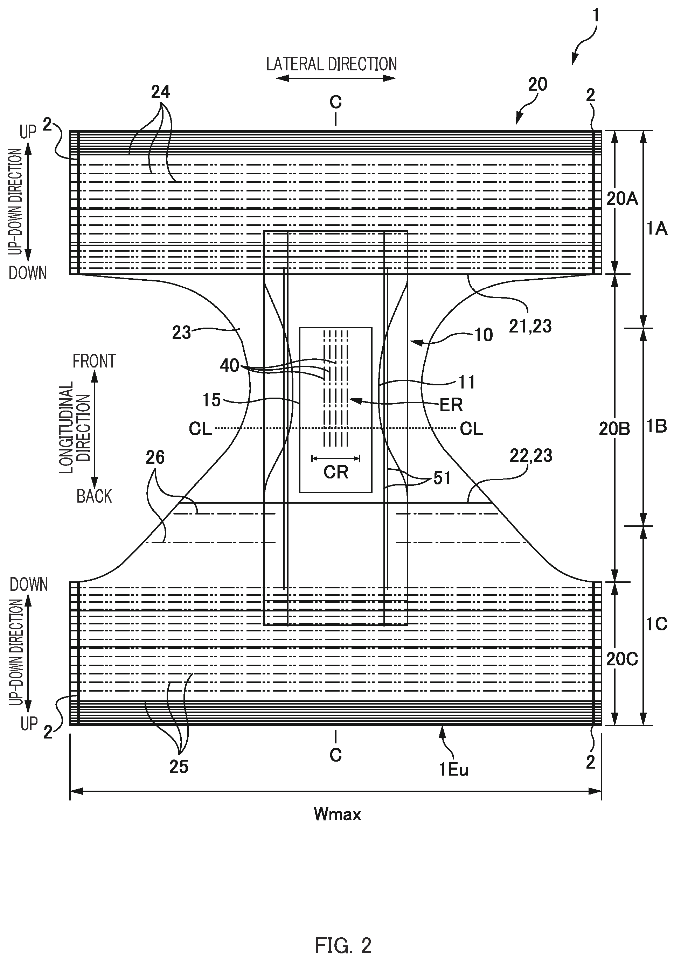

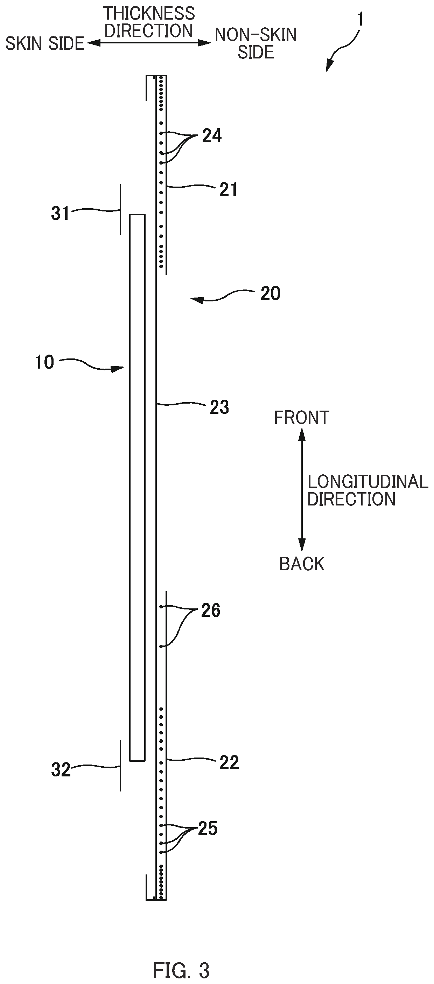

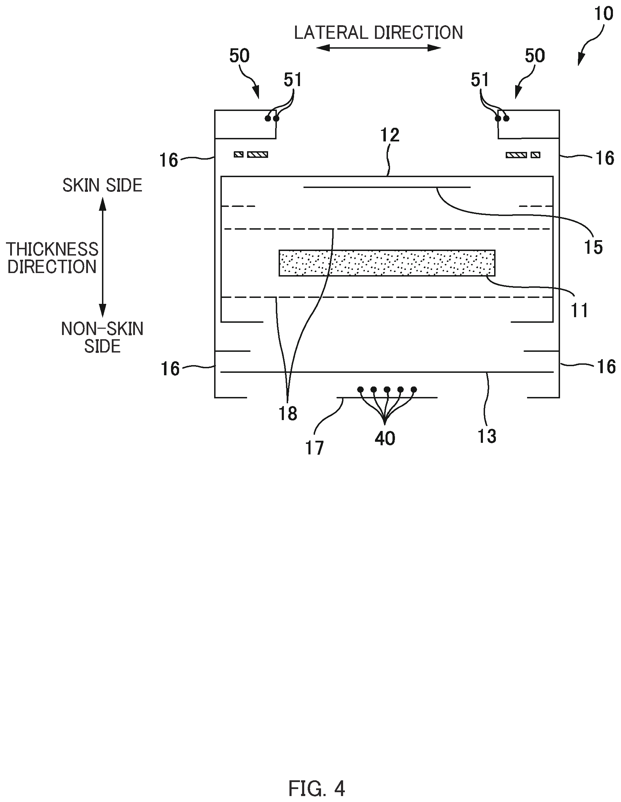

FIG. 1 is a schematic front view of the underpants-shaped disposable diaper 1. FIG. 2 is a schematic plan view of the diaper 1 in an unfolded and stretched state. FIG. 3 is a schematic cross-sectional view taken along the C-C line in FIG. 2. FIG. 4 is a schematic cross-sectional view of an absorbent main body 10 taken along a line CL-CL in FIG. 2. Here, the "unfolded state" of the diaper 1 refers to a state in which the diaper 1 is unfolded in the longitudinal direction and the diaper 1 is spread out flat by unjoining waist joining portions 2, which will be described later.

In the underpants-shaped state shown in FIG. 1, the diaper 1 has an up-down direction, a lateral direction, and a front-back direction that intersect one another, and includes a waist opening BH and a pair of leg openings LH. Also, in the unfolded state shown in FIG. 2, the diaper 1 has a longitudinal direction, a lateral direction, and a thickness direction that intersect one another. The longitudinal direction is a direction extending along the up-down direction of the diaper 1 in the underpants-shaped state. The thickness direction is a direction in which members constituting the diaper 1 are overlaid on each other (see FIG. 4). In the up-down direction, the waist opening BH side is the upper side, and the crotch side is the lower side. In the front-back direction, the abdominal side of the wearer is the front side, and the back side of the wearer is the back side. In the thickness direction, the side that comes into contact with the wearer's body (skin) is the skin side, and the side opposite thereto is the non-skin side.

The "stretched state" of the diaper 1 refers to a state in which, by stretching elastic members (e.g., waist elastic members 24 and 25 and leak-proof-wall elastic members 51) of the diaper 1, the entire diaper 1 is stretched to eliminate wrinkles, or more specifically, is stretched such that the dimensions of constituent members of the diaper 1 (e.g., a top sheet 12 and inner-layer sheet 23, both described later) match or are close to the dimensions of respective members on their own.

As shown in FIG. 3, the diaper 1 includes an absorbent main body 10 and an exterior member 20 located on the non-skin side with respect to the absorbent main body 10. The absorbent main body 10 and the exterior member 20 are joined together using an adhesive (not shown). Cover sheets 31 and 32 that cover two longitudinal end portions of the absorbent main body 10 from the skin side may be provided. This can suppress biting of the edge of the absorbent main body 10 into the wearer and opening of the absorbent main body 10.

In the present specification, as shown in FIG. 2, in the unfolded and stretched state of the diaper 1, when the diaper 1 is divided into three equal regions in the longitudinal direction, the front-side region is referred to as a "front-side portion 1A", the central region is referred to as a "crotch portion 1B", and the back-side region is referred to as a "back-side portion 1C". The diaper 1 in the unfolded state is folded one time at a longitudinal central position CL, and two lateral side portions of the exterior member 20 in the front-side portion 1A and two lateral side portions of the exterior member 20 in the back-side portion 1C are joined together, thereby forming the diaper 1 in the underpants-shaped state shown in FIG. 1. Examples of the joining method for the waist joining portion 2 may include welding or joining using an adhesive.

As shown in FIG. 4, the absorbent main body 10 includes: an absorbent core 11 that absorbs excreted fluid; a liquid-permeable top sheet 12 (for example, a nonwoven fabric sheet) that is arranged on the skin side in the thickness direction with respect to the absorbent core 11; a liquid-impermeable back sheet 13 (for example, a resin film such as polyethylene or polypropylene) that is arranged on the non-skin side in the thickness direction with respect to the absorbent core 11; a liquid-permeable intermediate sheet 15 (for example, a nonwoven fabric sheet) that is arranged between the top sheet 12 and the absorbent core 11; a pair of side sheets 16; and an elastic-member cover sheet 17. The elastic-member cover sheet 17 is a sheet member that covers, from the non-skin side, crotch elastic members 40 that stretch and contract in the longitudinal direction (details thereof will be described later).

The absorbent core 11 absorbs and retains excreted fluid such as urine. The surface of the absorbent core 11 may be covered with a liquid-permeable core-wrapping sheet 18 (such as tissue paper). Examples of the absorbent core 11 include an absorbent core obtained by shaping liquid-absorbent fibers such as pulp containing superabsorbent polymer (SAP) into a predetermined shape. The absorbent core 11 of the present embodiment has a shape in which the longitudinal central portion is constricted inward in the lateral direction; however, the planar shape of the absorbent core 11 is not particularly limited.

A pair of leak-proof-wall portions 50 extending in the longitudinal direction is provided on two lateral side portions of the absorbent main body 10. As shown in FIG. 4, in the side sheets 16, portions extending from two lateral side portions of the non-skin-side surface of the back sheet 13 are folded at a plurality of locations and are provided at two lateral side portions on the skin-side surface of the top sheet 12, thereby forming the leak-proof-wall portions 50. Leak-proof-wall elastic members 51 (for example, elastic strings) are attached to the leak-proof-wall portions 50 while being stretched along the longitudinal direction. Due to stretchability of the leak-proof-wall elastic members 51, the leak-proof-wall portions 50 rise toward the wearer's skin side and stand upright, thereby blocking excrement.

The exterior member 20 is a sheet member having a shape in which the longitudinal central portion is constricted inward in the lateral direction. As shown in FIG. 3, the exterior member 20 includes: a front-side outer-layer sheet 21; a back-side outer-layer sheet 22; an inner-layer sheet 23; waist elastic members 24 and 25; and buttocks elastic members 26.

Examples of the front-side outer-layer sheet 21, the back-side outer-layer sheet 22, and the inner-layer sheet 23 may include flexible sheet members such as nonwoven fabric sheets. The inner-layer sheet 23 is a sheet member that forms the outer shape of the exterior member 20 and extends continuously from the front-side portion 1A to the back-side portion 1C via the crotch portion 1B. The front-side outer-layer sheet 21 is arranged on the non-skin side with respect to the inner-layer sheet 23 and on a part of the front-side portion 1A. The front-side outer-layer sheet 21 overlaps the waist joining portion 2 in the longitudinal direction, and the longitudinal length of the front-side outer-layer sheet 21 is the same as that of the waist joining portion 2. The back-side outer-layer sheet 22 is arranged on the non-skin side with respect to the inner-layer sheet 23, and on a part of the crotch portion 1B and the back-side portion 1C. The back-side outer-layer sheet 22 overlaps the waist joining portion 2 in the longitudinal direction and extends toward the crotch side with respect to the waist joining portion 2. In the diaper 1 in the unfolded state, the front-side outer-layer sheet 21 and the back-side outer-layer sheet 22 are spaced apart in the longitudinal direction, and the inner-layer sheet 23 serving as the non-skin-side surface of the diaper 1 is exposed to the outside at a position between the front-side outer-layer sheet 21 and the back-side outer-layer sheet 22.

The waist elastic members 24 and 25 and the buttocks elastic members 26 are elastic members that stretch and contract in the lateral direction, and examples thereof may include string-like elastic members such as elastic strings. As shown in FIG. 2, the waist elastic members 24 and 25 overlap the waist joining portion 2 in the longitudinal direction. In the following description, the waist elastic members located in the front-side portion 1A are also referred to as front-side waist elastic members 24, and the waist elastic members located in the back-side portion 1C are also referred to as back-side waist elastic members 25. The buttocks elastic members 26 are located on the crotch side in the longitudinal direction (on the lower side in the up-down direction) with respect to the waist joining portion 2. In the present embodiment, there are two buttocks elastic members 26, but this number is not particularly limited.

In addition, the waist elastic members 24 and 25 and the buttocks elastic members 26 are arranged side by side in the up-down direction in the underpants-shaped state at a position between the inner-layer sheet 23 and the front-side outer-layer sheet 21 or the back-side outer-layer sheet 22, and are fixed while being stretched along the lateral direction. Therefore, the exterior member 20 fits the wearer's waist and buttocks.

### Crotch Elastic Members 40

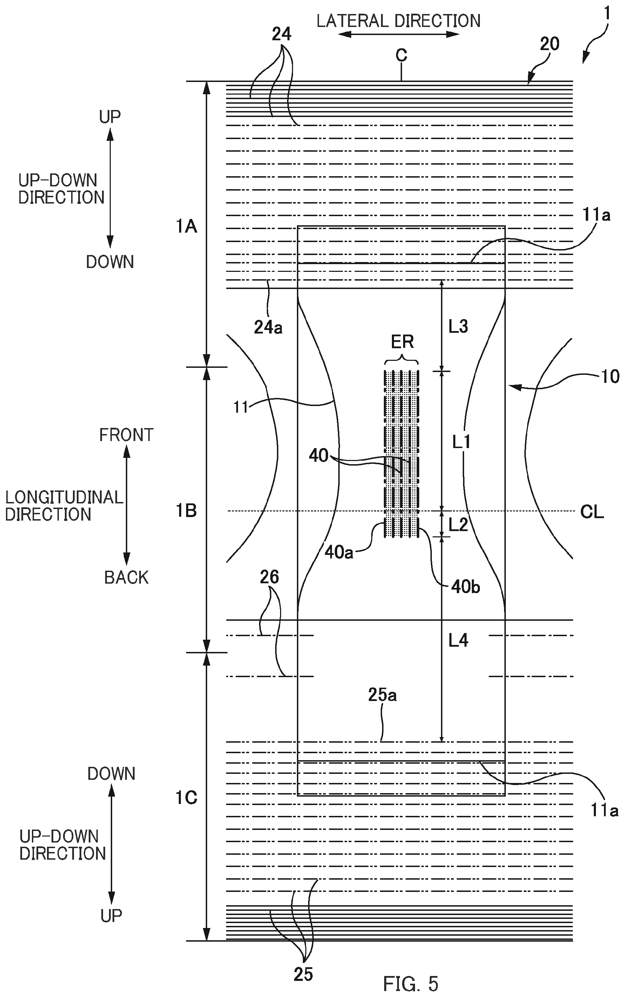

FIG. 5 is a view illustrating the arrangement of the crotch elastic members 40, and is a partial schematic plan view of the diaper 1 in the unfolded and stretched state. The diaper 1 includes the "crotch elastic members 40" that stretch and contract in the longitudinal direction. At least a part of the crotch elastic members 40 is provided at the lateral central portion of the "crotch portion 1B", which is a central region obtained by dividing the diaper 1 in the unfolded and stretched state into three equal regions in the longitudinal direction.

The diaper 1 of the present embodiment includes a plurality of string-like elastic members (for example, elastic strings) serving as the crotch elastic members 40. As shown in FIG. 4, the plurality of crotch elastic members 40 (five string-like elastic members) are arranged side by side at intervals in the lateral direction at a position between the back sheet 13 and the elastic-member cover sheet 17 in the thickness direction. Each of the crotch elastic members 40 is attached while being stretched along the longitudinal direction. In the drawings, only a stretchable portion of the crotch elastic members 40, that is, the portion of the crotch elastic members 40 that exhibits elasticity, is shown.

The crotch elastic members 40 that are arranged at the lateral central portion in the crotch portion 1B overlap the absorbent core 11 in the thickness direction. In addition, the crotch elastic members 40 are integrally joined to the absorbent core 11 with an adhesive or the like via the back sheet 13. Therefore, when the diaper 1 is worn, the crotch elastic members 40 contract in the longitudinal direction, and the absorbent core 11 is easily deformed so as to be pushed up toward the skin side and brought into close contact with the wearer's crotch portion. Specifically, the absorbent core 11 is likely to bend at a boundary between the lateral central portion where the crotch elastic members 40 are arranged and side portions located outward thereof in the lateral direction. In other words, the lateral central portion of the absorbent core 11 is likely to deform into a convex shape toward the upper side (toward the wearer's crotch portion). Therefore, a gap is unlikely to be formed between the wearer's crotch portion and the absorbent core 11, thereby suppressing leakage of excreted fluid such as urine along the wearer's skin to the outside of the diaper 1.

In the present specification, the region delimited by the crotch elastic members 40 is also referred to as a "crotch elastic region ER". Specifically, in the case where the crotch elastic members 40 serving as the plurality of string-like elastic members are arranged at intervals in the lateral direction, the crotch elastic region ER is defined as follows. The crotch elastic region ER refers to, in the crotch portion 1B, the region between a leftmost crotch elastic member 40a (string-like elastic member) and a rightmost crotch elastic member 40b (string-like elastic member), the region being enclosed by a virtual line connecting longitudinal front-side ends of the crotch elastic members 40 (stretchable portions) arranged in the lateral direction and a virtual line connecting longitudinal back-side ends of the crotch elastic members 40 (stretchable portions). In addition, the crotch elastic members 40 are not limited to the plurality of string-like elastic members, but may instead be a stretchy sheet (such as a stretchy nonwoven fabric sheet or a stretchy film) or a flat rubber (a strip-shaped rubber). In this case, the region in which the stretchy sheet is arranged is defined as the "crotch elastic region ER". In the case where a plurality of strip-shaped stretchy sheets or flat rubbers are arranged at intervals in the lateral direction, the same applies as in the case of the string-like elastic members. That is, the region between the rightmost crotch elastic member 40 (strip-shaped stretchy sheet or flat rubber) and the leftmost crotch elastic member 40 is defined as the crotch elastic region ER.

In addition, at least a part of the crotch elastic member 40 is simply required to be located in the crotch portion 1B. The crotch elastic member 40 may extend to the front-side portion 1A, may extend to the back-side portion 1C, or may be contained within the crotch portion 1B. The crotch elastic member 40 being located in the lateral central portion of the crotch portion 1B means that, as shown in FIG. 2, at least a part of the crotch elastic member 40 (crotch elastic region ER) is located in the central region CR obtained by dividing the absorbent core 11 into three equal regions at the maximum width of the absorbent core 11.

### Lifting Test

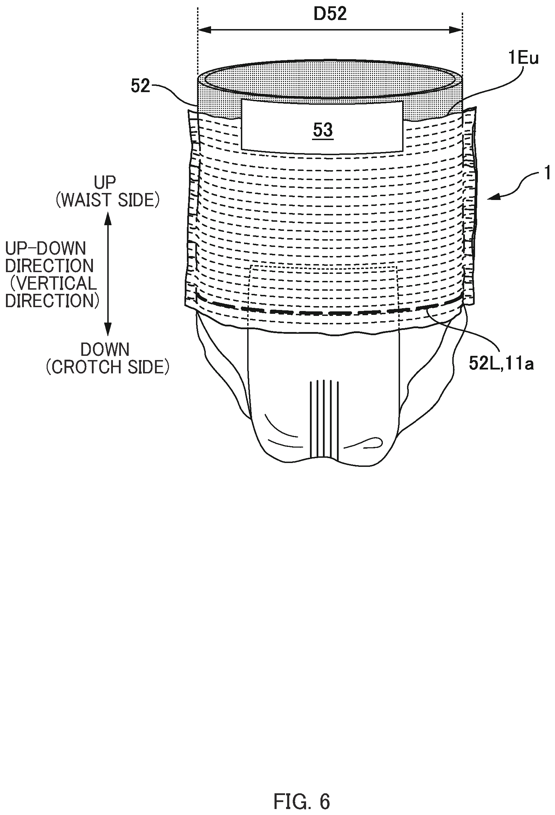

FIGS. 6, 7, and 8 are illustrative views of the lifting test. In the present embodiment, as shown in FIG. 4, at the longitudinal central position CL of the diaper 1 in the unfolded and stretched state, the absorbent core 11 is a single layer. The longitudinal central position CL of the diaper 1 is a position that comes into contact with the wearer's crotch portion. Therefore, compared to the case where a plurality of absorbent cores are stacked in the thickness direction at the longitudinal central position CL, the wearer is less likely to feel discomfort caused by stiffness (foreign-body sensation).

However, the absorbent core 11 need only have a single-layer structure at least at the longitudinal central position CL. Therefore, a multilayer structure in which a plurality of absorbent cores 11 are stacked may be provided at a position offset from the longitudinal central position CL. For example, an absorbent core composed of pulp may have thereon a so-called SAP sheet in which an SAP layer is attached to a hydrophilic sheet.

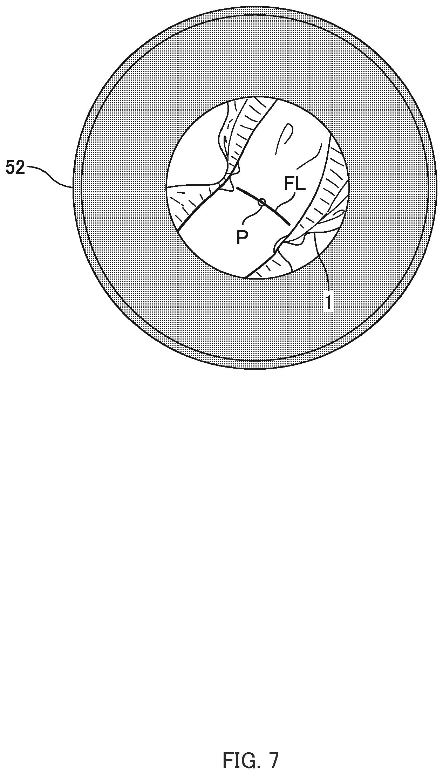

Furthermore, in the diaper 1 of the present embodiment, when a "lifting test" is performed, a lateral central portion 1Elc at a lower end 1El of the diaper 1 forms an upwardly convex shape. The lifting test is a test in which, after liquid is dropped onto a dropping position P that is a lateral central position of a fold position FL for folding the diaper 1 one time in the longitudinal direction (FIG. 7), two lateral end portions of the waist-side end portion of the diaper 1 in the longitudinally folded state are gripped and lifted. The amount of liquid (300 ml) corresponds to the amount of urine excreted in one to two urinations. The longitudinally folded state refers to the state in which the diaper 1 is folded one time with the skin-side surface facing inward.

In general, when an absorbent core absorbs excreted fluid, the entire lower end of the diaper in the worn state (in the longitudinally folded state) sags downward due to its weight. Specifically, the entire lower end of a region in which the absorbent core is arranged forms either a substantially straight line or a downwardly convex shape from the lateral side portions toward the central portion. As a result, the close contact of the absorbent core with the wearer's crotch portion is reduced, thereby making the wearer more likely to feel discomfort.

In contrast, in the diaper 1 of the present embodiment, after absorption of excreted fluid, the lateral central portion 1Elc at the lower end 1El of the diaper 1 in the worn state (in the longitudinally folded state) is pushed upward in the up-down direction of the diaper 1 relative to the side portions thereof. In other words, even after one to two excretions, the state in which the absorbent core 11 is in close contact with the wearer's crotch portion is maintained. As described above, according to the diaper 1 of the present embodiment, which takes into account deformation of the absorbent core 11 after excretion by using a lifting test, wearer's discomfort caused by sagging of the diaper 1 can be reduced. Furthermore, since a gap is unlikely to form between the wearer's crotch portion and the absorbent core 11, the absorbent core 11 can receive and absorb excreted fluid even when excretion occurs repeatedly. This can suppress leakage of excreted fluid along the wearer's skin.

As described above, the diaper 1 of the present embodiment includes the crotch elastic members 40. Thus, due to elastic force of the crotch elastic members 40, even after absorption of excreted fluid, the lateral central portion 1Elc at the lower end 1El of the folded diaper 1 is pushed toward the skin side, thereby forming an upwardly convex shape.

In addition, in the diaper 1 of the present embodiment, the absorbent core 11 is a single layer at the longitudinal central position CL (at a position substantially corresponding to the lower end 1El in the folded state). In the case where the absorbent core has a multilayer structure at the longitudinal central position and the widths of the plurality of layers differ, the absorbent core tends to bend at a position where the number of stacked layers changes. This may cause unintended deformation. In contrast, in the diaper 1 of the present embodiment, since a bending starting point due to a change in the number of stacked layers of the absorbent core 11 is not present at the longitudinal central position CL, the risk of unintended deformation can be reduced. Therefore, the lateral central portion 1Elc at the lower end 1El of the diaper 1 in the folded state can more reliably form an upwardly convex shape.

Here, the method of the "lifting test" will be described in detail.
(1) The diaper 1 to be measured is removed from a package. The diaper 1 is left to stand for 24 hours in a test room (at a room temperature of 20°C, a humidity of approximately 60%) while remaining in the state in which the diaper 1 is packaged in the package. In the case where, in the package, for example, two lateral side portions of the waist portion of the diaper 1 are folded inward or the diaper 1 is folded one time in the longitudinal direction, the diaper 1 is left to stand in the folded state for packaging.
(2) Thereafter, as shown in FIG. 6, the waist portion of the diaper 1 is mounted to a cylindrical jig 52 (a hollow cylindrical jig). In the case of the underpants-shaped diaper 1, the diaper 1 in the state where the waist joining portions 2 are joined together is mounted to the cylindrical jig 52. In the case of the tape-type diaper (not shown), the waist portion of the tape-type diaper is wound around the cylindrical jig 52 while the front waist portion and the back waist portion are joined together with a fastening tape.

In the case of an absorbent article intended for adults, the diameter (outer diameter) D52 of the cylindrical jig 52 is 210 mm. This length is obtained by dividing the circumferential length at approximately 63% elongation by π, where the circumferential length (inner circumferential length) of the waist opening BH of the diaper 1 of the present embodiment at maximum elongation is defined as 100%. In the case of an absorbent article intended for infants, the diameter (outer diameter) D52 of the cylindrical jig 52 is 165 mm. The thickness of the cylindrical jig 52 is approximately 5 mm. However, the inner diameter of the cylindrical jig 52 only needs to be large enough to allow a burette to be inserted therein and artificial urine to be dropped. The diaper 1 is set on the cylindrical jig 52 such that an upper end (waist-side end) 1Eu of the diaper 1 is aligned in a straight line. If necessary, the diaper 1 may be fixed to the cylindrical jig 52 using a tape 53. In addition, the diaper 1 is set such that a lower end 52L of the cylindrical jig 52 coincides with an upper end 11a (longitudinal end) of the absorbent core 11. When the length from the upper end 1Eu of the diaper 1 to the upper end 11a of the absorbent core 11 differs between the front side and the back side, the diaper 1 is set on the cylindrical jig 52 with reference to the shorter length (the upper end 11a of the absorbent core 11 located closer to the waist side).

Finally, in the state where the diaper 1 is mounted on the cylindrical jig 52, it is confirmed that the absorbent core 11 (or the crotch elastic members 40, when provided) is not stretched (i.e., is in a natural state). In the case where the absorbent core 11 or the crotch elastic member 40 is stretched, the diameter D52 of the cylindrical jig 52 is adjusted, a new diaper 1 is prepared, and the test is performed again. In the case where the intended wearer is neither an adult nor an infant, the diameter D52 of the cylindrical jig 52 is determined based on the assumed waist circumferential length of the intended wearer (for example, in the case of an underpants-shaped absorbent article, a cylindrical jig 52 is prepared based on a circumferential length when the waist portion is stretched to approximately 60% to 65% of its circumferential length in the maximum stretched state).

(3) Next, as shown in FIG. 7, when the skin-side surface of the diaper 1 is viewed from above the cylindrical jig 52, the dropping position P (lateral central position of the fold position FL for folding the diaper 1 in the longitudinal direction) is identified. In general, underpants-shaped and tape-type absorbent articles are each folded one time at the longitudinal central position CL and then packaged. In this case, the lateral central position of the fold position FL during packaging is defined as the dropping position P. Otherwise, the dropping position P refers to the longitudinal and lateral central position of the diaper 1 in the unfolded and stretched state.

(4) Next, artificial urine (300 ml in total) is dropped onto the dropping position P. The artificial urine is dropped onto the diaper 1 while its lower end portion is lifted off from a test table 50. The artificial urine was prepared by dissolving 200 g of urea, 80 g of sodium chloride, 8 g of magnesium sulfate, 3 g of calcium chloride, and approximately 1 g of dye: Brilliant Blue FCF (Blue No. 1) in 10 L of ion-exchange water. Specifically, 150 ml of artificial urine is dropped at a constant rate (150 ml/15 seconds) from a position 30 mm above the dropping position P (the skin-side surface of the diaper 1) using a burette. Three minutes later, 150 ml of artificial urine is dropped at a constant rate (150 ml/15 seconds) in the same manner.

(5) Next, three minutes after completion of the dropping of the artificial urine, the diaper 1 is removed from the cylindrical jig 52. Thereafter, two clips 51 (for example, bulldog clips) each having a gripping width W51 of 65 mm are prepared. In addition, a pair of horizontal support bars 54 extending horizontally from a vertical support rod (not shown) is prepared.

In the case of the underpants-shaped diaper 1 (see FIG. 1), marks M are respectively applied inwardly in the lateral direction from two lateral ends 1Eus, 1Eus of a waist-side end (upper end) 1Eu of the diaper 1 in its natural state over a length corresponding to the gripping width W51 of the clip 51 (65 mm). The marks M are provided at positions spaced downward by h51 (= 15 mm) from the waist-side end 1Eu of the diaper 1. That is, the marks M are provided along the lateral direction of the diaper 1, starting from the outer end of the waist joining portion 2 at positions spaced downward by h51 (= 15 mm) from the respective lateral ends 1Eus, 1Eus of the waist-side end 1Eu of the diaper 1.

In the case of the tape-type diaper, the lateral end positions at the upper end of the absorbent core in the front-side portion are used as references. Marks are applied outwardly in the lateral direction from the lateral end positions at the upper end of the absorbent core, over a length corresponding to the gripping width W51 (65 mm) of the clip 51. The marks M are provided along the lateral direction of the tape-type diaper, starting from positions spaced downward by h51 (= 15 mm) from the waist-side end of the tape-type diaper folded one time.

In addition, a length W1 from the left end of the left mark M to the right end of the right mark M on the diaper 1 in its natural state is measured using a ruler or the like. Then, with the lower end of each of the clips 51 aligned with the mark M, the clips 51 respectively grip two lateral end portions of the waist-side end portion (upper end portion) of the diaper 1 in the longitudinally folded state. Specifically, in the lateral direction of the diaper 1, the outer end of each mark M and the outer end at the lower end of each clip 51 are aligned with each other. The clips 51 grip the entire folded diaper 1 (both the front-side portion 1A and the back-side portion 1C). At this time, the clips 51 also grip the horizontal support bars 54 together with the diaper 1, on the back side of the diaper 1. If necessary, a slip-preventing member may be provided on the horizontal support bars 54 to prevent the position of the clip 51 from shifting relative to the diaper 1.

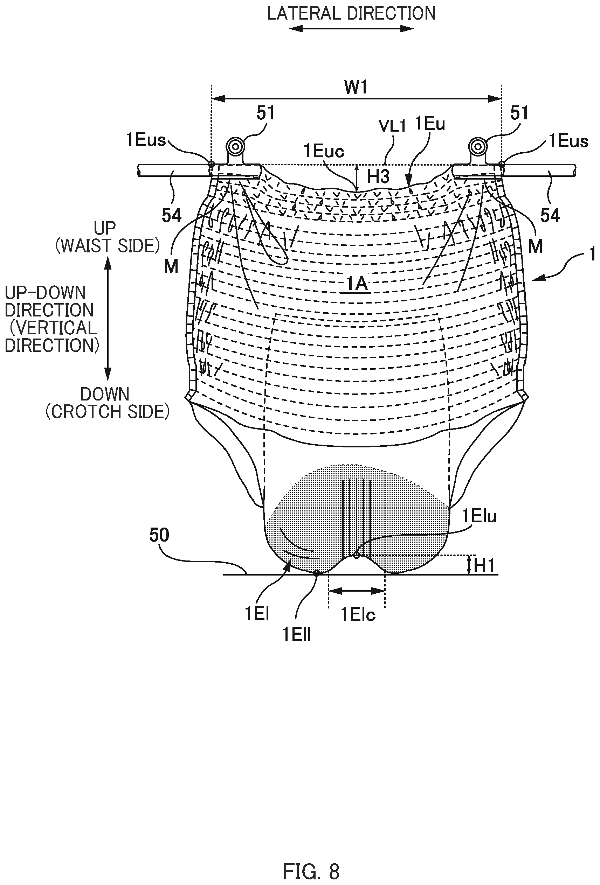

(6) Thereafter, as shown in FIG. 8, the clips 51 are lifted in the vertical direction such that the lateral spacing W1 between the left and right clips 51 (lower ends) is maintained at the spacing W1 in the diaper 1 in the natural state. That is, the diaper 1 is lifted in the state in which the waist portion is not stretched in the lateral direction (in the natural state). In addition, with the left and right clips 51 kept at the constant (horizontal) vertical height, the diaper 1 is lifted until a lowermost end 1Ell of the diaper 1 in the folded state comes into contact with the test table 50 (horizontal plane). Then, the diaper 1 is observed from the front-side portion 1A. Preferably, a still image of the diaper 1 is acquired using a digital camera. Then, it is confirmed that the lateral central portion 1Elc at the lower end 1El of the diaper 1 (specifically, the lower end of a portion where the absorbent core 11 is arranged) is positioned above the lateral side portions in the up-down direction (vertical direction). In the case where this is confirmed, it is determined that the lateral central portion 1Elc at the lower end 1El of the diaper 1 forms an upwardly convex shape.

In fact, the underpants-shaped diaper 1 of the present embodiment (the diaper 1 having the configuration shown in FIGS. 1 to 4) was prepared, and a lifting test was performed. Specifically, in the unfolded and stretched state of the diaper 1, the following diaper 1 was prepared and subjected to the test. The absorbent core 11 had: the longitudinal length of 480 mm; the maximum width of 200 mm; and the basis weight of 564 g/m², and the crotch elastic member 40 had: the length of 160 mm; the stretch ratio of 2.2 times; the fineness of 780 dtex; and the lateral pitch of 8 mm. Note that the stretch ratio is a value obtained by dividing the length of the crotch elastic member 40 at maximum elongation (in the stretched state of the diaper 1) by the length of the crotch elastic member 40 in its unloaded state (in the natural state of the diaper 1). As a result, in the diaper 1 of the present embodiment, the lateral central portion 1Elc at the lower end 1El in the folded state formed an upwardly convex shape.

Therefore, according to the diaper 1 of the present embodiment, the crotch elastic members 40 allow the absorbent core 11 to be brought into close contact with the wearer's crotch portion, thereby suppressing leakage of excreted fluid. In addition, it is possible to suppress discomfort caused by stiffness of the absorbent core 11 at the wearer's crotch portion. Furthermore, in the diaper 1 of the present embodiment, since deformation of the absorbent core 11 after excretion is also taken into consideration, discomfort caused by a decrease in close contact with the crotch portion after excretion can also be suppressed.

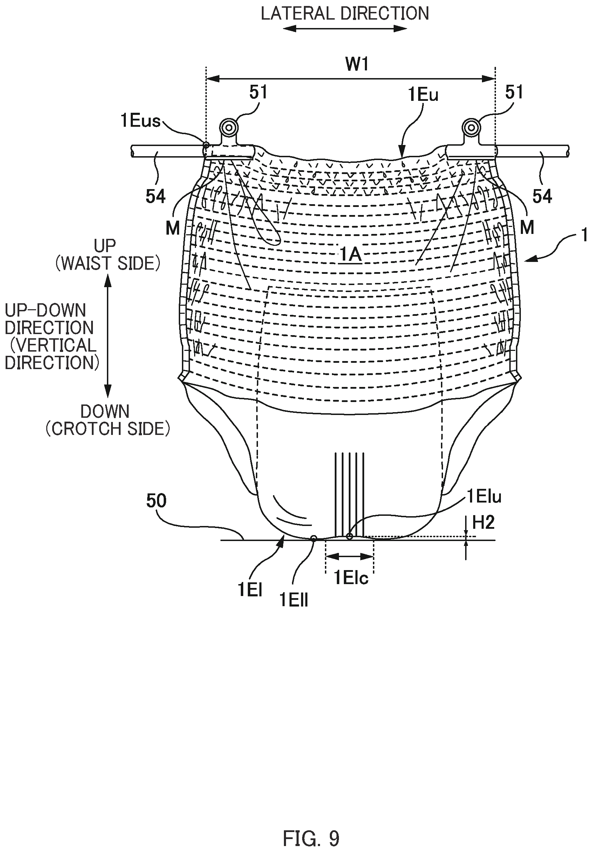

FIG. 9 is an illustrative view of a pre-absorption lifting test. In the case of performing the "pre-absorption lifting test" in which the two lateral end portions of the waist-side end portion of the diaper 1 that is in a dry state and a longitudinally folded state are gripped and lifted, it is preferable that the lateral central portion 1Elc at the lower end 1El of the diaper 1 forms an upwardly convex shape, as shown in FIG. 9. As a result, even before excretion, the absorbent core 11 comes into close contact with the wearer's crotch portion. Therefore, at the time of first excretion, the absorbent core 11 can receive and absorb excreted fluid, and leakage of excreted fluid along the wearer's skin can be suppressed.

Furthermore, it is preferable that a height H1 of the convex shape of the lateral central portion 1Elc at the lower end 1El of the diaper 1 in the aforementioned lifting test (FIG. 8) is greater than a height H2 of the convex shape of the lateral central portion 1Elc at the lower end 1El of the diaper 1 in the pre-absorption lifting test (FIG. 9) (H1 > H2).

As described above, after absorption of excreted fluid, even when the two lateral side portions of the absorbent core 11 sag due to the weight of excreted fluid, it is preferable that sagging of the lateral central portion is suppressed and that a state in which the lateral central portion is pushed up toward the skin side by the crotch elastic members 40 is maintained. As a result, discomfort of the wearer caused by sagging of the absorbent core 11 can be reduced. Furthermore, even when excretion occurs repeatedly, a gap is unlikely to be formed between the wearer's crotch portion and the absorbent core 11, thereby suppressing leakage of excreted fluid along the wearer's skin.

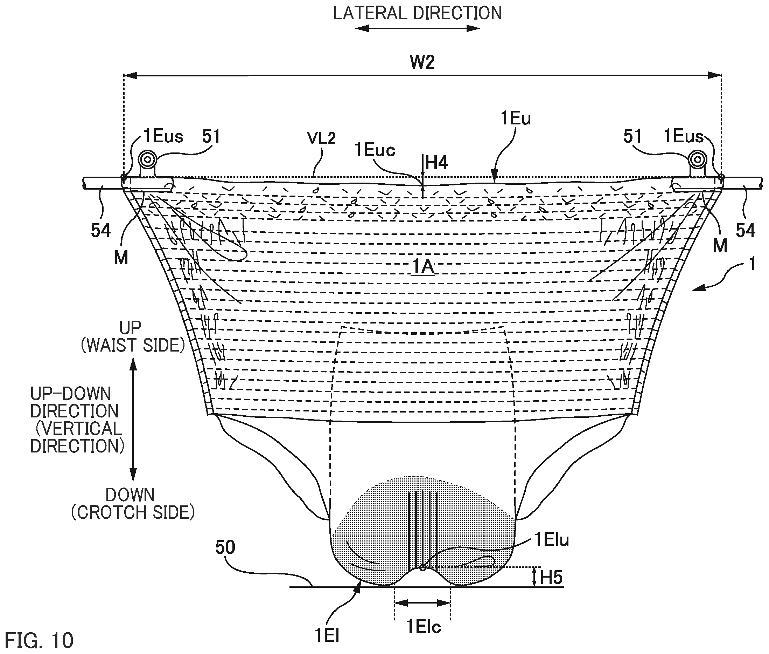

Note that the comparison between the heights H1 and H2 of the convex shapes may be obtained based on image data captured by a digital camera in the state where the lifting test and the pre-absorption lifting test are performed. Specifically, in the image data, the position of the uppermost end 1Elu of the lateral central portion 1Elc at the lower end 1El of the diaper 1 is identified, and the height from the test table 50 is determined. However, the present invention is not limited to the above configuration, and for example, the height may be directly measured using a ruler on the diaper 1 in the actually lifted state. A height H3 of a concave shape (see FIG. 8) and a height H4 from a virtual line VL2 to the waist-side end 1Eu (see FIG. 10), which are described later, can also be determined in the same manner.

Here, the method of the "pre-absorption lifting test" will be described. The method is the same as that of the lifting test, except for the step of dropping artificial urine onto the diaper 1. Briefly, first, a diaper 1 to be measured is taken out from a package. The diaper 1 is left to stand for 24 hours in a test room (at a room temperature of 20°C, a humidity of 60%) while remaining in the state in which the diaper 1 is packaged in the package. Next, if necessary, the fold of the packaged diaper 1 is unfolded, and the marks M are applied at positions of the diaper 1 to be gripped by the clips 51. Further, the spacing W1 between the left and right marks M is measured. Then, with the lower end of each of the clips 51 aligned with the mark M, the clips 51 respectively grip two lateral end portions at the waist-side end portion (upper end portion) of the diaper 1 that is folded one time in the longitudinal direction. Thereafter, with the left and right clips 51 kept at the constant (horizontal) vertical height, the clips 51 are lifted in the vertical direction until the lowermost end 1Ell of the diaper 1 in the folded state comes into contact with the test table 50 (horizontal plane). At this time, the diaper 1 is lifted in a natural state in which the waist portion does not stretch in the lateral direction (a state in which the spacing between the left and right clips 51 (lower ends) is maintained at W1). Then, the diaper 1 is observed from the front-side portion 1A.

In addition, in the case where the underpants-shaped diaper 1 is subjected to the lifting test (see FIG. 8), when the waist-side end 1Eu of the diaper 1 forms a concave shape that curves downward, it is preferable that the following condition holds. In the lifting test, it is preferable that the height H1 of the convex shape of the lower end portion of the diaper 1 is smaller than the height H3 of the concave shape of the waist-side end 1Eu of the diaper 1 (H1 < H3). Specifically, it is preferable that the height H1 of the convex shape of the lateral central portion 1Elc at the lower end 1El of the diaper 1 is smaller than the vertical length H3 from the virtual line VL1 connecting the left and right ends 1Eus, 1Eus at the waist-side end 1Eu of the diaper 1 to the lateral central portion 1Euc of the waist-side end 1Eu. The lateral central portion 1Euc at the waist-side end 1Eu of the diaper 1 is defined as a position corresponding to the lateral central portion of the virtual line VL1.

According to the above, after excretion, the lateral central portion 1Elc at the lower end of the diaper 1 forms a convex shape, while the height thereof is appropriately suppressed. Therefore, while the absorbent core 11 is brought into close contact with the wearer's crotch portion, it is possible to prevent an excessive pressing against the wearer's crotch portion. Thus, it is possible to suppress leakage of excreted fluid while reducing discomfort of the wearer.

FIG. 10 is a view of a state in which the waist portion is stretched after the lifting test. After the lifting test (FIG. 8) is performed on the underpants-shaped diaper 1, the gripping positions of the waist-side end portion of the diaper 1 are widened to a length W2, which is 80% of a maximum lateral length Wmax when the waist-side end 1Eu of the diaper 1 is maximally stretched in the lateral direction (= Wmax × 80%; see FIG. 2). In this state, it is preferable that the following condition holds. Specifically, the lateral length from the left end of the left clip 51 (lower end) that lifts the diaper 1 to the right end of the right clip 51 (lower end) is widened to the length W2, 80% of the maximum lateral length.

In the above, it is preferable that a vertical length H4 from the virtual line VL2 connecting the left and right ends 1Eus, 1Eus at the waist-side end 1Eu of the diaper 1 to the lateral central portion 1Euc of the waist-side end 1Eu is smaller than a height H5 of the convex shape of the lateral central portion 1Elc at the lower end 1El of the diaper 1 (H4 < H5). The "height of the convex shape" at this time refers to the height H5 of the convex shape at the lower end 1El in the state where the waist portion is stretched after the lifting test, and is not the height H1 of the convex shape at the lower end 1El when the diaper 1 in the natural state is lifted (FIG. 8). In addition, the lateral central portion 1Euc at the waist-side end 1Eu of the diaper 1 is defined as a position corresponding to the lateral central portion of the virtual line VL2.

According to the above, in the worn state in which the waist portion of the diaper 1 is stretched, the difference in the vertical direction (up-down direction of the diaper 1) between the lateral side portions and the central portion at the waist-side end 1Eu is reduced. Therefore, the diaper 1 can uniformly cover the entire waist of the wearer. For example, it is possible to suppress exposure of the wearer's abdomen due to sagging of the lateral central portion at the waist-side end portion of the diaper 1. On the other hand, the lateral central portion 1Elc at the lower end 1El of the diaper 1 forms an upwardly convex shape, and the height H5 thereof is sufficiently ensured. Therefore, even after excretion, the absorbent core 11 can be brought into close contact with the wearer's crotch portion, thereby reducing the wearer's discomfort caused by sagging of the diaper 1.

In FIG. 10, the waist-side end 1Eu of the diaper 1 has a downwardly curved shape; however, the waist-side end 1Eu may extend along the lateral direction (that is, H4 may be zero), or the waist-side end 1Eu may be curved in an upwardly convex shape.

In addition, in the unfolded and stretched state of the diaper 1 (FIG. 5), it is preferable that a length L1 of a stretchable portion of the crotch elastic members 40 located on the front side with respect to the longitudinal central position CL of the diaper 1 is longer than a length L2 of a stretchable portion of the crotch elastic members 40 located on the back side with respect to the longitudinal central position CL of the diaper 1 (L1 > L2).

The urethral opening of the wearer (particularly the urethral opening of a male wearer) is located on the abdominal side (front side) with respect to a position directly below the crotch. Therefore, as described above, it is preferable that the stretchable portion of the crotch elastic members 40 is located so as to be biased toward the front side of the diaper 1 in the longitudinal direction. As a result, the crotch elastic members 40 are likely to be located at a position corresponding to the urethral opening, and the crotch elastic members 40 can directly push up the absorbent core 11 toward the urethral opening. Thus, the absorbent core 11 is likely to be brought into close contact with the urethral opening, thereby allowing the absorbent core 11 to receive and absorb excreted fluid. This can further suppress leakage of excreted fluid.

In addition, in the unfolded and stretched state of the diaper 1 (FIG. 5), it is preferable that the stretchable portion of the crotch elastic members 40 is arranged so as to span the longitudinal central position CL of the diaper 1.

As a result, in the lifting test, the crotch elastic members 40 are arranged at a position serving as the lower end 1El of the diaper 1 in the folded state. Therefore, even after the absorbent core 11 absorbs excreted fluid (artificial urine), the crotch elastic members 40 can directly push up the absorbent core 11 at the longitudinal central position. Accordingly, in the lifting test, the lateral central portion at the lower end 1El of the diaper 1 can form an upwardly convex shape. In addition, the longitudinal central position CL of the diaper 1 is likely to be located directly below the wearer's crotch or slightly on the back side. The urethral opening of the wearer (particularly a male wearer) is located on the abdominal side with respect to the position directly below the crotch; however, when the wearer is in a standing position or in a supine sleeping posture, excreted fluid discharged from the urethral opening is likely to flow toward the back side. Even in this case, according to the above-described configuration, the absorbent core 11 is in close contact with the portion directly below the wearer's crotch and on the back side thereof. Thus, the absorbent core 11 can absorb excreted fluid, and leakage of excreted fluid can be further suppressed.

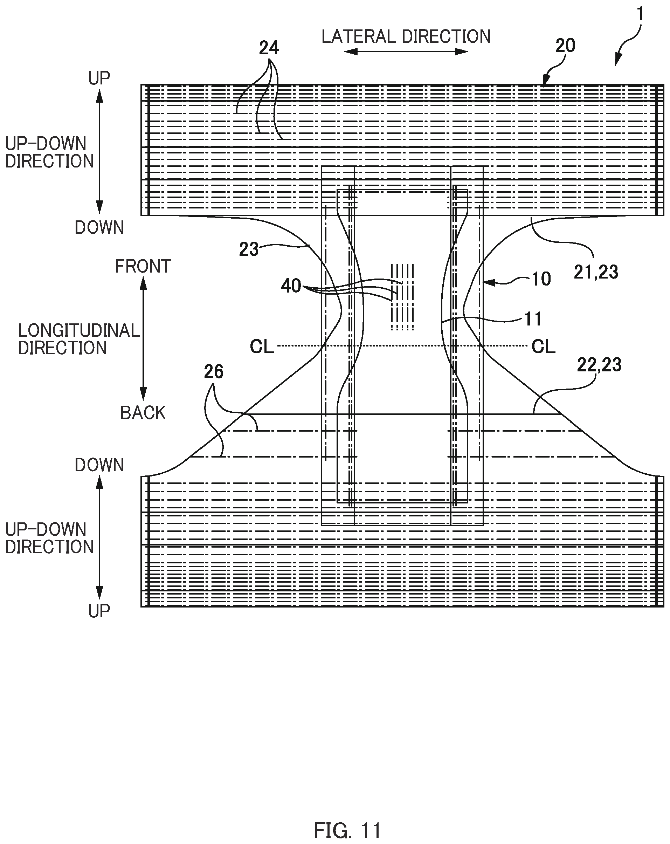

FIG. 11 is an illustrative view of diaper 1 according to a modified example, and is a schematic plan view of the diaper 1 in the unfolded and stretched state. The present invention is not limited to the above. In the lifting test, as long as the lateral central portion 1Elc at the lower end 1El of the diaper 1 forms an upwardly convex shape, the crotch elastic members 40 need not span the longitudinal central position CL of the diaper 1. That is, the crotch elastic members 40 may be arranged on the front side (see FIG. 11) or the back side (not shown) with respect to the longitudinal central position CL of the diaper 1. In this case as well, in conjunction with deformation of the absorbent core 11 pushed up by the crotch elastic members 40 on the front side or the back side with respect to the central position CL, the absorbent core 11 at the central position CL is also pushed up. In such a case, in the lifting test, the lateral central portion 1Elc at the lower end 1El of the diaper 1 can form an upwardly convex shape.

The diaper 1 of the present embodiment includes the waist elastic members 24 and 25 and the buttocks elastic members 26 which serve as "lateral elastic members" that stretch and contract in the lateral direction. In the drawings, only the stretchable portions (portions that exhibit elasticity) of the elastic members included in the diaper 1, namely the waist elastic members 24 and 25 and the buttocks elastic members 26, are shown. In this case, in the unfolded and stretched state of the diaper 1 (FIG. 5), when the diaper 1 is viewed in the thickness direction, it is preferable that the aforementioned crotch elastic region ER (the region delimited by the crotch elastic member 40) does not overlap each of the stretchable portions of the waist elastic members 24 and 25 and the buttocks elastic members 26.

As a result, it is possible to prevent the longitudinal elastic force of the crotch elastic members 40 from being inhibited by the lateral elastic force of the waist elastic members 24 and 25 and the buttocks elastic members 26. Therefore, before excretion, the close contact of the absorbent core 11 with the wearer's crotch portion is enhanced, thereby further suppressing leakage of excreted fluid. Furthermore, even after the absorbent core 11 absorbs excreted fluid (artificial urine), the crotch elastic members 40 can push up the absorbent core 11. Thus, in the lifting test, the lateral central portion 1Elc at the lower end 1El of the diaper 1 can have an upwardly convex shape.

The diaper 1 of the present embodiment includes a plurality of the waist elastic members 24 and 25 serving as "continuous elastic members" that stretch and contract in the lateral direction and are continuous at positions overlapping the absorbent core 11 with respect to the lateral direction (that is, are continuous from a position corresponding to the left end of the absorbent core 11 to a position corresponding to the right end of the absorbent core 11). The continuous elastic members may overlap or need not overlap the absorbent core 11 as viewed in the thickness direction.

In this case, in the unfolded and stretched state of the diaper 1, out of the continuous elastic members, a continuous elastic member closest to the crotch elastic members 40 on the front side in the longitudinal direction is referred to as a front-side continuous elastic member (here, a lowermost front-side waist elastic member 24a), and a continuous elastic member closest to the crotch elastic members 40 on the back side in the longitudinal direction is referred to as a back-side continuous elastic member (here, a lowermost back-side waist elastic member 25a). At this time, it is preferable that, in the longitudinal direction, a length L3 from the front-side end of the stretchable portion of the crotch elastic member 40 to the front-side continuous elastic member (the lowermost front-side waist elastic member 24a) is shorter than a length L4 from the back-side end of the stretchable portion of the crotch elastic member 40 to the back-side continuous elastic member (the lowermost back-side waist elastic member 25a) (L3 < L4).

As a result, on the front side of the diaper 1, in the longitudinal direction, the length (L3) of the region between the waist elastic member 24 and the crotch elastic member 40 is reduced. That is, the length (L3) of the region where the elastic members and the absorbent core 11 do not overlap in the thickness direction, and where the force of the elastic members does not directly act on the absorbent core 11, is reduced. Therefore, on the front side of the diaper 1, the absorbent core 11 is more likely to be brought into close contact with the wearer than on the back side. Accordingly, the absorbent core 11 is likely to be brought into close contact with the wearer's urethral opening located on the front side, and can receive and absorb excreted fluid. This can further suppress leakage of excreted fluid.

On the other hand, on the back side of the diaper 1, the length (L4) of the region between the waist elastic members 25 and the crotch elastic member 40 in the longitudinal direction is increased. Therefore, the degree of close contact of the absorbent core 11 with the wearer's buttocks is appropriately reduced. Accordingly, a space is formed between the wearer's buttocks and the absorbent core 11, and this space can temporarily accommodate feces or a large amount of excreted urine. Thus, it is possible to suppress overflow of excrement from the diaper 1.

The buttocks elastic members 26 of the present embodiment are cut (discontinuous) at a position overlapping the absorbent core 11 in the thickness direction. However, the present invention is not limited thereto, and the buttocks elastic members 26 may continuously extend from the left end to the right end of the absorbent core 11. In this case, the buttocks elastic members 26 also correspond to the continuous elastic members.

In addition, the buttocks elastic members 26 of the present embodiment are arranged linearly along the lateral direction. However, the present invention is not limited thereto, and an elastic member arranged along the lower outer edge of the back-side outer-layer sheet 22 may be provided on the diaper 1. That is, at or below the lower end portion of the waist joining portion 2, an elastic member having: a portion that is inclined downward from two lateral side portions toward the lateral central portion of the back-side outer-layer sheet 22; and a portion that extends in the lateral direction at the lateral central portion of the back-side outer-layer sheet 22 may be provided on the diaper 1. This elastic member also has a portion that stretches and contracts in the lateral direction and corresponds to the lateral elastic member; therefore, it is preferable that the elastic member does not overlap the crotch elastic region ER. In addition, when this elastic member is continuous at a position overlapping the absorbent core 11 in the lateral direction, it corresponds to the continuous elastic member.

Furthermore, when the crotch elastic members 40 are a plurality of string-like elastic members (for example, elastic strings), the following configuration is preferable. In the unfolded and stretched state of the diaper 1 (FIG. 5), the continuous elastic member closest to the crotch elastic members 40 on the back side in the longitudinal direction is defined as a back-side continuous elastic member (here, the lowermost back-side waist elastic member 25a). The back-side continuous elastic member may overlap or need not overlap the absorbent core 11 as viewed in the thickness direction. At this time, it is preferable that a force required to stretch the stretchable portion of the back-side continuous elastic member (the lowermost back-side waist elastic member 25a) by a predetermined length in the lateral direction is smaller than an average value of forces required to stretch the respective crotch elastic members 40 (the plurality of string-like elastic members) by a predetermined length in the longitudinal direction.

In the above case, due to the relatively weak elastic force of the back-side continuous elastic member (back waist elastic member 25a), the degree of close contact of the absorbent core 11 with the wearer's buttocks is appropriately reduced. Accordingly, a space is formed between the wearer's buttocks and the absorbent core 11, and feces or a large amount of excreted urine can be temporarily accommodated in this space. Therefore, it is possible to suppress overflow of excrement from the diaper 1. On the other hand, due to the relatively strong elastic force of the crotch elastic members 40, the close contact of the absorbent core 11 with the wearer's crotch portion is enhanced. This can further suppress leakage of excreted fluid. In addition, even after excretion, the absorbent core 11 can be pushed up toward the skin side. Therefore, in the lifting test, the lateral central portion 1Elc at the lower end 1El of the diaper 1 can form an upwardly convex shape.

The elastic forces of the crotch elastic members 40 and the back-side continuous elastic members (the forces required to stretch them by a predetermined length) can be measured using a tensile tester (for example, one manufactured by Instron Corporation (Model: 5965) or equivalent thereof).

First, the crotch elastic member 40 and the back-side continuous elastic member are cut out from the target diaper 1. In the case where the crotch elastic member 40 includes a plurality of string-like elastic members, each of the string-like elastic members is cut out. Samples are prepared by adjusting the cut-out crotch elastic member 40 and the back-side continuous elastic member to have the same length.

Next, both end portions of each sample in the tensile direction thereof are fixed by a pair of chucks of the tensile tester. In the case of the crotch elastic member 40, the longitudinal direction of the diaper 1 corresponds to the tensile direction. In the case of the back-side continuous elastic member, the lateral direction of the diaper 1 corresponds to the tensile direction. A gripping width of the sample by the chucks is kept constant, and an initial distance between the chucks is adjusted such that the sample does not slacken or slackens only slightly. Next, the distance between the pair of chucks is increased in the stretching direction of the sample at a constant speed (e.g., 100 mm/min), to a predetermined length or to a length longer than the predetermined length. The measured value (unit: N) obtained when the sample is stretched by a predetermined length is defined as a force required to stretch the sample by the predetermined length. An average value of the measured values of the samples of the crotch elastic members 40 (five string-like elastic members, in the present embodiment) is calculated. The average value is compared with the measured value of the back-side continuous elastic member, and it is then confirmed that the measured value of the back-side continuous elastic member is smaller.

In addition, it is preferable that, at the longitudinal central position CL of the diaper 1, a low-basis-weight region in which the basis weight (g/m²) of the absorbent core 11 is lower than that in the surrounding region is not present. The low-basis-weight region also includes a slit in which the basis weight of the absorbent core 11 is zero. That is, it is preferable that the basis weight of the absorbent core 11 is substantially constant at the longitudinal central position CL.

With this configuration, even when the absorbent core 11 has a single-layer structure at the longitudinal central position CL, the absorbent core 11 can ensure the absorption amount of excreted fluid. Thus, leakage of excreted fluid can be suppressed. In addition, the low-basis-weight region may serve as a starting point for bending of the absorbent core 11. Therefore, in the diaper 1 of the present embodiment, the low-basis-weight region that may serve as a starting point of bending is not present at the longitudinal central position CL, and thus the risk of unintended deformation can be reduced. Accordingly, the lateral central portion 1Elc at the lower end 1El of the diaper 1 in the state being folded one time can more reliably form an upwardly convex shape. The absence of the low-basis-weight region in the absorbent core 11 can be confirmed by a known method. For example, it is preferable to, using an electron microscope or the like, enlarge and observe a cross section obtained by cutting the absorbent core 11 at the central position CL and to confirm that the number of fiber cross-sections and the number of SAPs are substantially constant in the lateral direction.

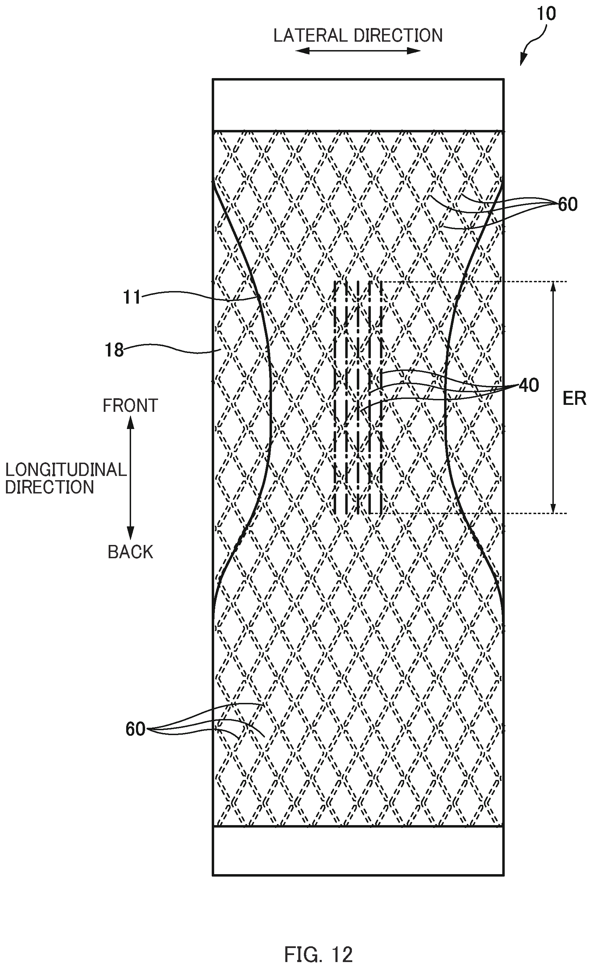

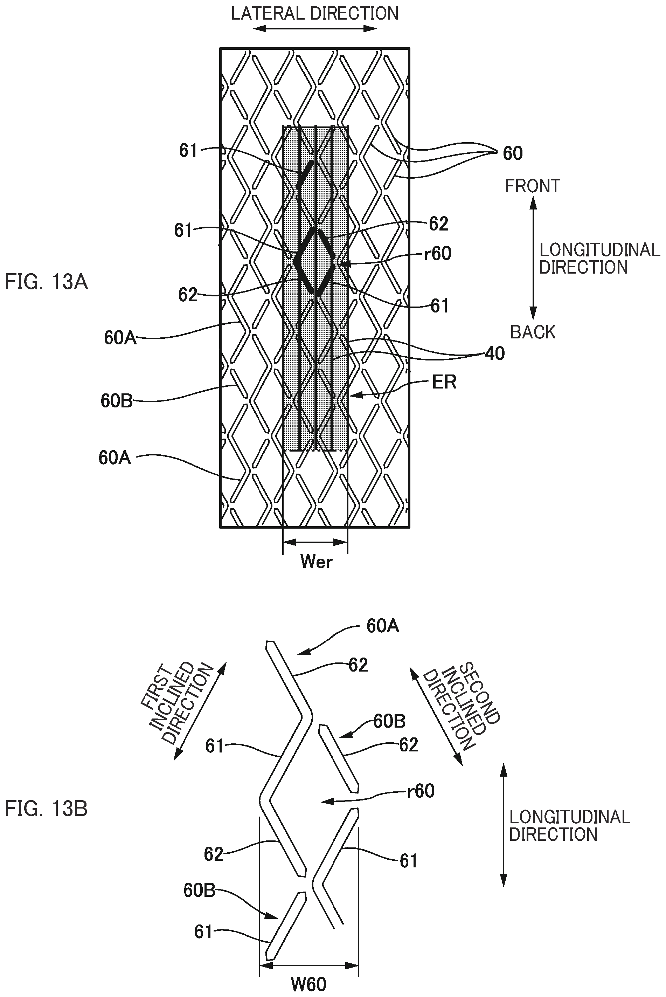

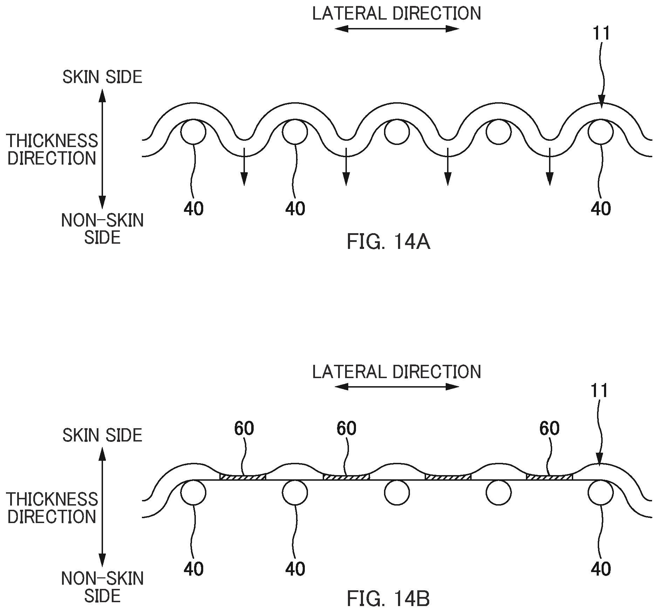

FIGS. 12, 13A, and 13B are illustrative views of compressed portions 60. FIGS. 14A and 14B are schematic cross-sectional views of the absorbent core 11. Note that FIGS. 14A and 14B are illustrated with materials such as the back sheet 13 omitted, for the sake of simplifying the drawings.

The diaper 1 includes a plurality of line-like compressed portions 60 in which the absorbent core 11 is compressed in the thickness direction. Specifically, in the "compressed portions" of the present embodiment, the absorbent core 11 and the core-wrapping sheet 18 are compressed in the thickness direction by embossing. The compressed portions 60 are provided over the entire planar region of the absorbent core 11 and the core-wrapping sheet 18. Due to the compression, the fiber density of the absorbent core 11 is higher than that of a non-compressed portion, and the thickness of the absorbent core 11 is reduced. The compressed portions 60 are simply required to be in a state in which at least a part of the absorbent core 11 is compressed in the thickness direction. Therefore, the compressed portions are not limited to the above, and may be compressed portions formed by compressing in the thickness direction from the skin-side surface of the top sheet 12 to the absorbent core 11, or may be compressed portions formed by compression from the non-skin side of the absorbent core 11.

The compressed portions 60 of the present embodiment are formed in a line-like manner. The line-like compressed portions 60 may be formed, for example, by a group of a plurality of dot-shaped (circular) compressed portions, or may be continuous groove-shaped compressed portions. Specifically, as shown in FIG. 13A, substantially Z-shaped compressed portions 60A and straight-line compressed portions 60B are alternately arranged in the longitudinal direction. A plurality of rows of the compressed portions 60 are arranged in the lateral direction. Each row of the compressed portions 60 has a shape extending in the longitudinal direction while meandering in the lateral direction (a zigzag shape). In rows of the compressed portions 60 adjacent to each other in the lateral direction, the positions of the substantially Z-shaped compressed portions 60A are shifted in the longitudinal direction. Therefore, the compressed portions 60 are arranged such that, as a whole, they appear as a diagonal lattice pattern. However, an arrangement pattern of the compressed portions 60 is not limited to the illustrated pattern. In addition, either dot-shaped or line-like compressed portions may be formed discretely.

It is preferable that, when the crotch elastic members 40 are a plurality of string-like elastic members, the compressed portions 60 are arranged between the plurality of crotch elastic members 40 (string-like elastic members) arranged in the lateral direction, as shown in FIG. 13A. The compressed portions 60 are simply required to be arranged in at least one of a plurality of regions between the crotch elastic members 40 adjacent to each other in the lateral direction (in the present embodiment, four regions between the five string-like elastic members). In addition, at least a part of the line-like or dot-shaped compressed portions 60 is simply required to be arranged between the crotch elastic members 40 adjacent to each other in the lateral direction.

FIG. 14A is a view illustrating the case in which the compressed portions 60 are not arranged between the crotch elastic members 40 (string-like elastic members). In this case, since the absorbent core 11 between the crotch elastic members 40 is not compressed, the absorbent core 11 has lower stiffness and is more easily deformed compared to the case in which the absorbent core 11 is compressed. Therefore, after excretion, the absorbent core 11 between the crotch elastic members 40 is likely to sag slightly due to the weight of absorbed liquid. In contrast, as shown in FIG. 14B, when the compressed portions 60 are arranged between the crotch elastic members 40 (string-like elastic members), stiffness of the absorbent core 11 between the crotch elastic members 40 is increased. Therefore, even after excretion, in conjunction with the absorbent core 11 directly pushed up by the crotch elastic members 40, the absorbent core 11 between the crotch elastic members 40 is also likely to be pushed up. Accordingly, the absorbent core 11 can be brought into close contact with the wearer's crotch portion in a surface-contact manner, thereby reducing wearer's discomfort caused by a decrease in close contact. Furthermore, a gap between the wearer's crotch portion and the absorbent core 11 is further reduced, thereby further suppressing leakage of excreted fluid through the gap.

As shown in FIG. 13A, it is preferable that, in the crotch elastic region ER (the region delimited by the crotch elastic members 40), the compressed portion 60 has at least a part of inclined compressed portions 61 and 62 extending along a direction inclined relative to the longitudinal direction and the lateral direction. In FIG. 13A, some of the inclined compressed portions 61 and 62 located in the crotch elastic region ER are indicated by black filling.

Specifically, as shown in FIG. 13B, the substantially Z-shaped compressed portion 60A has a first inclined portion 61 extending along a first inclined direction and a pair of second inclined portions 62 extending from both ends of the first inclined portion 61 in opposite directions along a second inclined direction intersecting the first inclined direction. In addition, the plurality of straight-line compressed portions 60B are divided into the first inclined portion 61 extending along the first inclined direction and the second inclined portions 62 extending along the second inclined direction. That is, the inclined compressed portions include a plurality of the first inclined portions 61 and a plurality of the second inclined portions 62. In the crotch elastic region ER, the plurality of first inclined portions 61 and the plurality of second inclined portions 62 are arranged.

According to the above, the inclined compressed portions 61 and 62 serve as the starting points for bending, making the absorbent core 11 overlapping the crotch elastic region ER likely to deform. Therefore, in the lifting test, the lateral central portion 1Elc at the lower end 1El of the diaper 1 can form an upwardly convex shape. Furthermore, compared to the case where the compressed portions 60 extend along the longitudinal direction, a contractive force of the crotch elastic members 40 in the longitudinal direction is less likely to be inhibited, making the crotch elastic members 40 more likely to push up the absorbent core 11. Compared to the case where the compressed portions 60 extend along the lateral direction, deformation in which the absorbent core 11 overlapping the crotch elastic region ER curves into an upwardly convex shape is less likely to be inhibited.

As shown in FIG. 13A, the plurality of first inclined portions 61 are arranged at intervals in the second inclined direction, and the plurality of second inclined portions 62 are arranged at intervals in the first inclined direction. Therefore, as shown in FIG. 13B, a plurality of substantially diamond-shaped regions r60, each surrounded on four sides by two first inclined portions 61 and two second inclined portions 62 (compressed portions 60) are formed in the absorbent core 11. In this case, in the unfolded and stretched state of the diaper 1, it is preferable that a lateral length W60 of the region r60 delimited by the plurality of first inclined portions 61 and the plurality of second inclined portions 62 is shorter than a lateral length Wer of the crotch elastic region ER (W60 < Wer).

The lateral length W60 of the region r60 is the length from the leftmost position to the rightmost position of the inclined compressed portions 61 and 62 surrounding the region r60. In addition, the inclined compressed portions 61 and 62 that define the region r60 may be partially spaced apart, as in the present embodiment, or may be continuous.

With the above configuration, the substantially diamond-shaped region r60 (one unit of diagonal lattice patterns) is more likely to be located within a portion of the absorbent core 11 overlapping the crotch elastic region ER. Therefore, the inclined compressed portions 61 and 62 that define the region r60 serve as the starting points for bending, making the absorbent core 11 overlapping the crotch elastic region ER likely to deform. Therefore, in the lifting test, the lateral central portion 1Elc at the lower end 1El of the diaper 1 can form an upwardly convex shape. In addition, while the absorbent core 11 is folded, the portion of the absorbent core 11 within the region r60 is more likely to come into close contact, in a surface-contact manner, with the crotch portion of the wearer.

The diaper 1 of the present embodiment includes: the absorbent main body 10 including the absorbent core 11; and the exterior member 20. As shown in FIG. 2, it is preferable that the exterior member 20 has: a pair of waist portions 20A and 20C; and a crotch portion 20B that connects the pair of waist portions 20A and 20C. In the exterior member 20, the pair of waist portions 20A and 20C are portions that cover the wearer's waist. In the case of the underpants-shaped diaper 1 of the present embodiment, in the exterior member 20, portions overlapping the waist joining portion 2 with respect to the longitudinal direction are defined as the waist portions 20A and 20C.

In the above case, the exterior member 20 continuously extends from the front-side waist portion 20A to the back-side waist portion 20C via the crotch portion 20B. Therefore, in the crotch portion 1B of the diaper 1, the non-skin-side surface of the absorbent main body 10 is not exposed and is covered with the exterior member 20. Accordingly, the absorbent core 11 (absorbent main body 10) that has increased in weight after excretion, is pulled upward by the crotch portion 20B of the exterior member 20 that is continuous from the waist portions 20A and 20C. Thus, discomfort of the wearer caused by sagging of the absorbent core 11 can be reduced. The pair of waist portions 20A and 20C and the crotch portion 20B may be a single continuous sheet member, or may be formed by joining separate sheet members.

### Others

The above-described embodiment is intended to facilitate the understanding of the present invention and is not intended to limit the interpretation of the present invention. The present invention can be modified or improved without departing from the spirit thereof, and additionally, it is needless to say that equivalents thereof are included in the present invention.

### [REFERENCE SIGNS LIST]

1: underpants-shaped disposable diaper (absorbent article)
2: waist joining portion
10: absorbent main body
11: absorbent core, 12: top sheet
13: back sheet, 15: intermediate sheet
16: side sheet, 17: elastic-member cover sheet 20: exterior member
21: front-side outer-layer sheet, 22: back-side outer-layer sheet
23: inner-layer sheet
24: (front-side) waist elastic member (lateral elastic member, continuous elastic member)
25: (back-side) waist elastic member (lateral elastic member, continuous elastic member)
26: buttocks elastic member (lateral elastic member)
31: cover sheet, 32: cover sheet
40: crotch elastic member
50: test table, 51: clip
52: cylindrical jig, 53: tape
60: compressed portion
61: first inclined portion (inclined compressed portion)
62: second inclined portion (inclined compressed portion)

## Claims

1. An absorbent article having, in an unfolded and stretched state, a longitudinal direction, a lateral direction, and a thickness direction that intersect one another,
the absorbent article comprising:
an absorbent core; and
a crotch elastic member that stretches and contracts in the longitudinal direction,
at least a part of the crotch elastic member being provided in a lateral central portion of a crotch portion,
the crotch portion being a central region obtained by dividing the absorbent article into three equal regions in the longitudinal direction,
at a longitudinal central position of the absorbent article, the absorbent core being a single layer,
when a lifting test is performed, a lateral central portion at a lower end of the absorbent article forming an upwardly convex shape,
the lifting test being a test in which, after liquid is dropped onto a dropping position that is a lateral central position of a fold position for folding the absorbent article one time in the longitudinal direction, two lateral end portions of a waist-side end portion of the absorbent article in a longitudinally folded state are gripped and lifted.

2. The absorbent article according to claim 1, wherein
in an unfolded and stretched state,
a length of a stretchable portion of the crotch elastic member located on a front side with respect to the longitudinal central position of the absorbent article is longer than a length of a stretchable portion of the crotch elastic member located on a back side with respect to the longitudinal central position.

3. The absorbent article according to claim 1 or 2, wherein
in an unfolded and stretched state,
the stretchable portion of the crotch elastic member is arranged so as to span the longitudinal central position of the absorbent article.

4. The absorbent article according to claim 1 or 2, wherein
the absorbent article includes a lateral elastic member that stretches and contracts in the lateral direction,
in an unfolded and stretched state,
a region delimited by the crotch elastic member is referred to as a crotch elastic region, and
when the absorbent article is viewed in the thickness direction, the crotch elastic region does not overlap a stretchable portion of the lateral elastic member.

5. The absorbent article according to claim 1 or 2, wherein
the absorbent article includes a plurality of continuous elastic members that stretch and contract in the lateral direction and are continuous at a position overlapping the absorbent core with respect to the lateral direction,
in an unfolded and stretched state,
the continuous elastic member closest to the crotch elastic member on the front side in the longitudinal direction is referred to as a front-side continuous elastic member,
the continuous elastic member closest to the crotch elastic member on the back side in the longitudinal direction is referred to as a back-side continuous elastic member, and
in the longitudinal direction, a length from a front-side end of the stretchable portion of the crotch elastic member to the front-side continuous elastic member is shorter than a length from a back-side end of the stretchable portion of the crotch elastic member to the back-side continuous elastic member.

6. The absorbent article according to claim 1 or 2, wherein
the absorbent article includes a continuous elastic member that stretches and contracts in the lateral direction and is continuous at a position overlapping the absorbent core with respect to the lateral direction,
the crotch elastic member comprises a plurality of string-like elastic members,
in an unfolded and stretched state,
the continuous elastic member closest to the crotch elastic member on the back side in the longitudinal direction is referred to as a back-side continuous elastic member, and
a force required to stretch a stretchable portion of the back-side continuous elastic member by a predetermined length in the lateral direction is smaller than an average value of a force required to stretch each of the plurality of string-like elastic members by a predetermined length in the longitudinal direction.

7. The absorbent article according to claim 1 or 2, wherein
at the longitudinal central position of the absorbent article, a low-basis-weight region in which a basis weight of the absorbent core is lower than that in a surrounding region is not present.

8. The absorbent article according to claim 1 or 2, wherein
the absorbent article includes a compressed portion in which the absorbent core is compressed in the thickness direction,
the crotch elastic member comprises a plurality of string-like elastic members, and
the compressed portion is arranged between the plurality of string-like elastic members arranged in the lateral direction.

9. The absorbent article according to claim 1 or 2, wherein
the absorbent article includes a compressed portion in which the absorbent core is compressed in the thickness direction, and
in an unfolded and stretched state,
a region delimited by the crotch elastic member is referred to as a crotch elastic region, and
in the crotch elastic region, the compressed portion includes at least a part of an inclined compressed portion extending along a direction inclined relative to the longitudinal direction and the lateral direction.

10. The absorbent article according to claim 9, wherein
the inclined compressed portion includes a plurality of first inclined portions and a plurality of second inclined portions,
the plurality of first inclined portions extending along a first inclined direction,
the plurality of second inclined portions extending along a second inclined direction that intersects the first inclined direction,
the plurality of first inclined portions are arranged at intervals in the second inclined direction,
the plurality of second inclined portions are arranged at intervals in the first inclined direction, and
in an unfolded and stretched state, a lateral length of a region delimited by the plurality of first inclined portions and the plurality of second inclined portions is shorter than a lateral length of the crotch elastic region.

11. The absorbent article according to claim 1 or 2, wherein
when a pre-absorption lifting test is performed, a lateral central portion at a lower end of the absorbent article forms an upwardly convex shape,
the pre-absorption lifting test being a test in which the two lateral end portions of the waist-side end portion of the absorbent article that is in a dry state and a longitudinally folded state are gripped and lifted, and
a height of the convex shape in the lifting test is greater than a height of the convex shape in the pre-absorption lifting test.

12. The absorbent article according to claim 1 or 2, wherein
the absorbent article is an underpants-shaped absorbent article including a waist elastic member that stretches and contracts in the lateral direction,
when the lifting test is performed,
a waist-side end of the absorbent article forms a recessed shape downwardly curved, and
a height of the convex shape is smaller than a vertical length from a virtual line connecting left and right ends at the waist-side end of the absorbent article to a lateral central portion of the waist-side end.

13. The absorbent article according to claim 1 or 2, wherein
the absorbent article is an underpants-shaped absorbent article including a waist elastic member that stretches and contracts in the lateral direction, and
after the lifting test is performed, in a state in which a gripping position of the waist-side end is widened to a length that is 80% of a length when the waist-side end of the absorbent article is maximally stretched in the lateral direction,
a vertical length from a virtual line connecting left and right ends at the waist-side end of the absorbent article to a lateral central portion of the waist-side end is smaller than a height of the convex shape.

14. The absorbent article according to claim 1 or 2, wherein
the absorbent article includes an absorbent main body including the absorbent core and an exterior member, and
the exterior member includes a pair of waist portions and a crotch portion that connects the pair of waist portions.
